# EUROPÄISCHE PATENTSCHRIFT

(11) **EP 0 987 239 B1**
(45) Veröffentlichungstag und Bekanntmachung des Hinweises auf die Patenterteilung: **29.10.2003**
(21) Anmeldenummer: 99116199.3
(22) Anmeldetag: 24.08.1999
(51) Int. Cl.: C07C 29/00

(54) **Verfahren zur Synthese alpha-substituierter Ringsysteme**
Process for the preparation of alpha-substituted ringsystems
Procédé pour la préparation de systèmes cycliques alpha substitués

(30) Priorität: 03.09.1998 DE 19840107
(43) Veröffentlichungstag der Anmeldung: 22.03.2000
(73) Patentinhaber: Bayer Aktiengesellschaft, 51368 Leverkusen (DE)
(72) Erfinder: Gürtler, Christoph, Dr., 50676 Köln (DE); Jautelat, Manfred, Dr., 51399 Burscheid (DE)

(56) Entgegenhaltungen:
- K. HAMMER AND K. UNDHEIM: "Synthesis of Conformationally Restricted Serine Derivatives Through Ruthenium (II)-Catalyzed Ring Closing Metathesis" TETRAHEDRON, Bd. 53, Nr. 16, - 1997 Seiten 5925-5936, XP004105625 Great Britain
- M.T. CRIMMINS AND B.W. KING: "An Efficient Asymmetric Approach to Carbocyclic Nucleosides: Asymmetric Synthesis of 1592U89, a Potent Inhibitor of HIV Reverse Transcriptase" J. ORG. CHEM., Bd. 61, 1996, Seiten 4192-4193, XP002123495
- R. H. GRUBBS ET AL: "Ring-Closing Metathesis and related Processes in Organic Synthesis" ACCOUNTS OF CHEMICAL RESEARCH, Bd. 28, Nr. 11, - 1995 Seiten 437-476, XP002124360
- M. D. E. FORBES ET AL: "Solvent-Free Cyclization of Linear Dienes Using Olefin Metathesis and the Thorpe-Ingold Effect" J. AM. CHEM. SOC., Bd. 114, 1992, Seiten 10978-10980, XP002124361

## Beschreibung

Nach dem erfindungsgemäßen Verfahren ist es möglich, mit wenigen Reaktionsschritten einen neuen Zugang zu α-substituierten Ringsystemen zu schaffen, die gegebenenfalls weitere Substituenten haben. Cyclohexen-2-ol ist ein herausragendes Beispiel für eine erfindungsgemäß herstellbare Verbindung. Es ist ein wichtiges Zwischenprodukt für z.B. die Herstellung von Nylon® bzw. Dralon® (aus Cyclohexanon und ε-Caprolactam) und für die Synthese von Feinchemikalien.

Aus Cyclohexen-2-ol kann durch katalytische Isomerisierung, d. h. frei von weiteren Neben- oder Folgeprodukten, Cyclohexanon und durch Hydrierung Cyclohexanol gewonnen werden (sog. KA-Öl, das als Vorstufe z.B. für Nylon® und Dralon® Verwendung findet). Darüber hinaus ist durch katalytische Dehydrierung Phenol und durch katalytische Dehydratisierung Cyclohexadien zugänglich.

Cyclohexen-2-ol wurde bislang durch die selektive Hydrierung von Phenol erhalten. Dieses Verfahren besteht aus einer Vielzahl von Stufen bzw. Teilschritten. So muß aus Benzol zunächst Phenol hergestellt werden, üblicherweise durch das sog. Cumol-Verfahren, bei dem aus Benzol im Rahmen einer Friedel-Crafts Alkylierung Isopropylbenzol erhalten wird. Nachteilig bei diesem Schritt ist der hohe Aufwand, da dieser Schritt nicht katalytisch durchführbar ist und aus der Friedel-Crafts Alkylierung entsprechend große Mengen an z.B. Eisensalzen anfallen, die entweder entsorgt oder aufgearbeitet werden müssen. Anschließend wird unter Zuhilfenahme von Sauerstoff dieses Produkt in Phenol und Aceton umgelagert. Bei diesem Verfahren fällt grundsätzlich als Koppelprodukt Aceton an. Das auf diese Weise gewonnene Phenol wird einer selektiven Hydrierung unterworfen, die auf der Stufe des Cyclohexen-2-ols angehalten wird. Das Verfahren ist daher aufwendig, liefert große Mengen schwer rezyklierbarer Abfallprodukte und darüber hinaus das in nicht allen Fällen gewünschte Nebenprodukt Aceton. Eine Übersicht geben z.B. K. Weissermel und H.-J. Arpe in "Industrielle Organische Chemie", 4. Auflage, 1994, VCH Verlagsgesellschaft Weinheim, S. 375-379.

Es bestand also der Bedarf, ein Verfahren zu entwickeln, welches die Herstellung von α-substituierten Ringsystemen wie Cyclohexen-2-ol aus leicht zugänglichen Edukten bei gleichzeitiger Kostenreduktion verbessert.

Als gangbarer Weg für die Synthese von Cyclohexen-2-ol sowie weiteren α-substituierten Cycloolefinen mit dieser oder größeren Ringgröße könnte prinzipiell die Olefinmetathese angesehen werden (eine Beschreibung dieses Reaktionstyps geben z.B. M. Schuster, S. Blechert, *Angew. Chem.* **1997,** *109,* 2124 und S. Armstrong, *J*. *Chem. Soc., Perkin Trans. 1,* **1998**, 371). Die als Ausgangsprodukte benötigten Diene können leicht durch die sogenannte Telomerisationsreaktion oder auf anderen an sich bekannten Reaktionswegen zur Verfügung gestellt werden.

Durch Olefinmetathese fünktionalisierter terminaler Diene können verschiedene Produkte katalytisch leicht gewonnen werden, indem ein Ringschluß des Diens induziert wird, wobei bei dieser Reaktion Ethylen als weiteres Wertprodukt gewonnen wird. Aus dem Stand der Technik sind jedoch keine allgemein anwendbaren Angaben zur Durchführung bestimmter Synthesen zu entnehmen.

Das erfindungsgemäße Verfahren basiert auf Edukten, die bevorzugt aus einer Telomerisationsreaktion gewonnen werden können (T. Prinz, W. Keim, B. Drießen-Hölscher, *Angew. Chem*. **1996,** *108,* 1835; siehe auch z.B. K. Kaneda, H. Kurosaki, M. Terasawa, T. Imanaka, S. Teranishi, *J. Org. Chem.* **1981,** *46,* 2356; R. M. Manyik, K. E. Atkins, W. E. Walker, *J. Chem. Soc. D*. **1971,** *7,* 330 und z.B.: K. Kaneda, H. Kurosaki, M. Terasawa, T. Imanaka, S. Teranishi, *J. Org. Chem.* **1981,** *46*, 2356; R. M. Manyik, K. E. Atkins, W. E. Walker, *J. Chem. Soc. D.* **1971,** *7*, 330). Für diese sind auch alternative Synthesewege beschrieben worden oder leicht konzipierbar. Die Edukte sind leicht zugänglich, zum Beispiel durch Umsetzung eines Nucleophils wie Wasser oder Ammoniak mit gegebenenfalls substituierten Butadienen. Somit ist auch der Zugang zu anderen α-substituierten Cycloolefinen neben Cyclohexen-2-ol möglich.

Auf dem Gebiet der Cycloolefinierung durch Metathese war bereits die Synthese einer Cyclopentenoleinheit in einem komplexen Naturstoffvorläufermolekül bekannt, in welcher unter folgenden Bedingungen folgende Struktur synthetisiert wurde (M. T. Crimmins, B. W. King, *J. Org Chem.* **1996,** 4192):

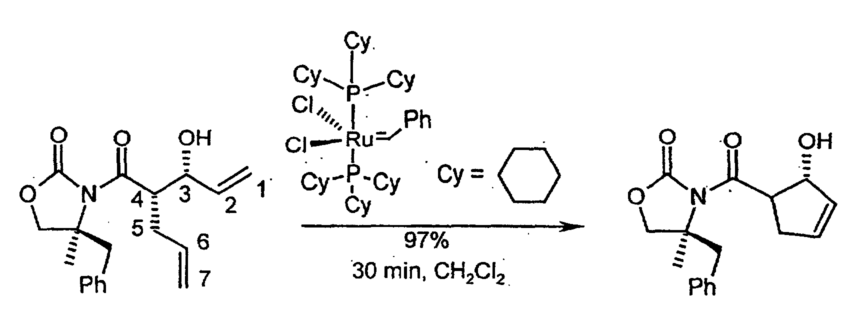

Die angegebenen Reaktionsbedingungen lassen sich aber nicht auf die Synthese von 2-Olefinen mit größeren Ringen übertragen. Es zeigte sich, daß für die Synthese von Cyclohexenolbausteinen als Vorstufen für die Synthese von optisch reinen Aminosäuren andere Bedingungen notwendig waren. Die Bedingungen unterscheiden sich zudem beim Wechsel vom S- zum R-Enantiomeren durch die benötigte Reaktionsdauer (K. Hammer, K. Undheim, *Tetrahedron* **1997,** 53, *5925).* Das eingesetzte Lösungsmittel Benzol mußte zum Erreichen der angegebenen Ausbeute (88 bzw. 89 %) in großen Mengen zugegeben werden. Somit wird in sehr großer Verdünnung mit einem gewerblich in diesen Mengen nicht mehr verwendbaren Lösungsmittel gearbeitet. Das Verfahren verwendet eine um den Faktor 5 bis 10 höhere Verdünnung als das erfindungsgemäße Verfahren.

Es stellte sich heraus, daß Benzol für die Synthese des Cyclohexenols ohnehin nicht geeignet ist, da keine vollständige Umsetzung eintritt. Die maximale Ausbeute unter Anwendung der geschilderten Bedingungen betrug 60 %. Als Nebenreaktion wurde die Umlagerung zum Keton (Isomerisierung Allylalkohol - Keton) beobachtet. Diese trat unter den Bedingungen und Lösungsmitteln, die ebenda für die Bildung des Fünfringes (in Dichlormethan) oder Siebenringes (Dichlorethan) angegeben wurden, verstärkt auf.

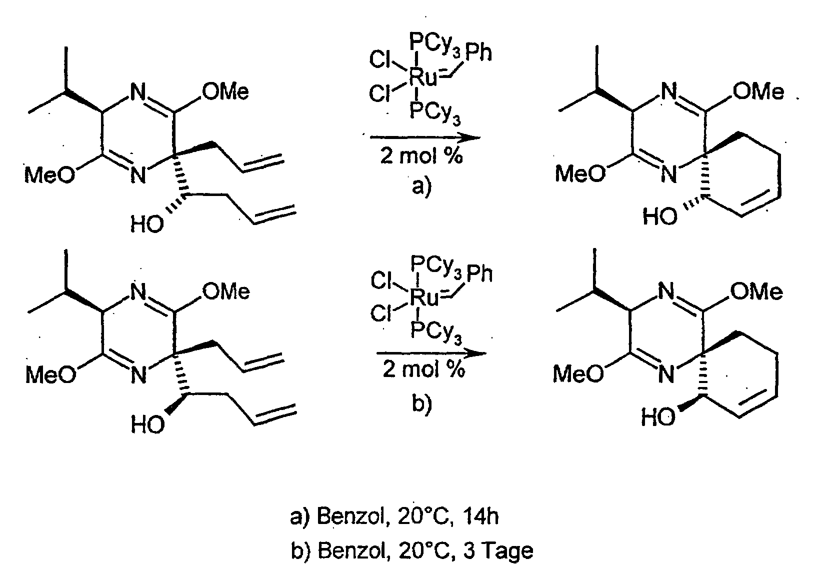

Die Übertragung des obigen Beispiels auf die Synthese von nicht wie oben gearteten Sechs- oder größeren Ringen ist somit nicht möglich.

In Hinsicht auf Verbindungen mit ungeschützten funktionellen Gruppen, wie sie im obigen Fall des 3-Hydroxy-1,7-octadiens vorliegt, kann aus den in der Literatur bekannten Reaktionsbedingungen nicht auf die für die oben beschriebenen Reaktion geeignete Reaktionsbedingungen geschlossen werden.

Daher lag der vorliegenden Erfindung auch die Aufgabe zugrunde, ein universell anwendbares Verfahren bereitzustellen, mit dem außer Cyclohexen-2-ol auch andere, gegebenenfalls größere, α-funktionalisierte ungesättigte Ringsysteme der Formel (II) erhältlich sind.

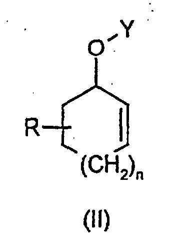

Hierin bedeuten jeweils unabhängig voneinander
- Y: Wasserstoff, Acyl, Alkyl, Aryl und Sulfonyl,
- R: Wasserstoff,
bedeutet
und
- n: die Zahlen 1, 2, 3 oder 4, bevorzugt 1 oder 2, besonders bevorzugt 1.

Überraschenderweise wurde gefunden, daß die Wahl des Lösungsmittels im erfindungsgemäßen Verfahren kritisch für den Erfolg der Reaktion ist.

Es zeigt sich, daß es in Abhängigkeit von der Wahl des Lösungsmittels zu Nebenreaktionen kommen kann. Diese Nebenreaktionen bestehen darin, daß eine Isomerisierung des Allylalkohols zum korrespondierenden Keton eintritt oder eine Oxidation zum entsprechenden Enon-System (siehe folgendes Reaktionsschema). Diese Nebenreaktionen lassen sich nur in wenigen Lösungsmitteln gut unterdrücken. In manchen Lösungsmitteln wie z.B. 1,2-Dichlorethan oder 1,1,1-Trichlorethan oder auch Dichlormethan treten diese Nebenreaktionen beim Versuch der Synthese der erfindungsgemäß herzustellenden Ringsysteme fast quantitativ auf. Für das erfindungsgemäße Verfahren prinzipiell geeignet sind z.B. Phenylessigsäureethylester oder auch superkritisches CO₂, wobei jedoch die Reaktionsraten besonders im letzteren Fall sehr langsam sind und somit für eine kommerzielle Anwendung weniger bevorzugt geeignet erscheinen.

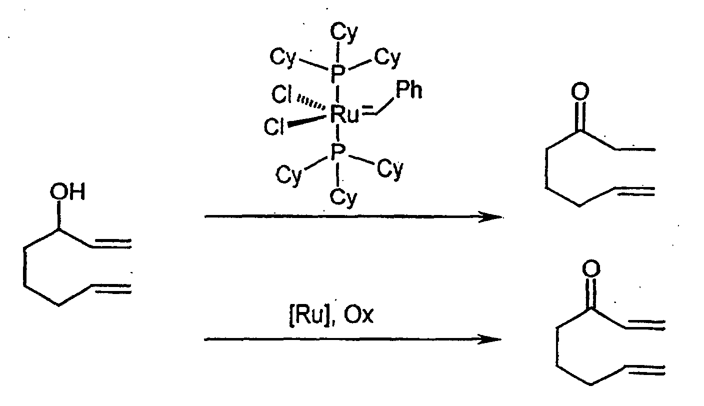

Die Nutzung der im folgenden aufgeführten Lösungsmittel ist also essentiell. Die Verwendung von sekundären Alkoholen oder tertiären Alkoholen als Lösungsmittel für das erfindungsgemäße Verfahren gestatten Ausbeuten von 50 % bis 80 %, ohne daß allerdings diese Nebenreaktionen vollständig unterdrückt werden können. Besonders bevorzugt ist die Verwendung von Trihalogenmethanverbindungen wie Chloroform oder Bromoform als Lösungsmittel für die Durchführung der erfindungsgemäßen Metathesereaktion, d.h. zur Erzeugung von Ringen einer Gliederzahl >5. Bevorzugt setzt man nur ein Lösungsmittel und kein Gemisch ein.
Die Durchführung dieser Reaktion in Substanz ist nicht möglich.

Der Ringschluß in der Olefinmetathesereaktion des erfindungsgemäßen Verfahrens wird mit einem Edelmetallkatalysator, bevorzugt einem Rutheniumkatalysator herbeigeführt. Als Katalysatoren werden im erfindungsgemäßen Verfahren besonders bevorzugt die in WO-A-93/20111 beschriebenen Ruthenium-Alkylidenverbindungen, die von A. W. Stumpf, E. Saive, A. Deomceau und A. F. Noels in *J. Chem. Soc., Chem. Commun.* **1995,** 1127-1128 beschriebenen Katalysatorsysteme auf Rutheniumbasis oder die von P. Schwab, R. H. Grubbs und J. W. Ziller in *J. Am*. *Chem. Soc*. **1996,** *118,* 100 veröffentlichten Katalysatorsysteme verwandt. Bis(tricyclohexylphosphin)benzylidenruthenium(IV)dichlorid ist ein besonders bevorzugter Katalysator. In einer bevorzugten Ausführungsform des erfindungsgemäßen Verfahrens wird permanent unter Schutzgasatmospäre gearbeitet, so daß das Katalysatorsystem über mehrere Zyklen eingesetzt werden kann.

Die Reaktionszeit ist variabel und hängt von der Reaktionstemperatur, dem Reaktionsdruck und der Art und der Menge des Katalysators ab. Normalerweise beträgt die Reaktionszeit 0.01 bis 30 Stunden, bevorzugt 1 bis 10 Stunden.

Der im erfindungsgemäßen Verfahren anzuwendende Druck ist kein kritischer Parameter. Es können auch Unterdrücke bis beispielsweise 0.1 bar und Überdrücke bis beispielsweise 100 bar verwandt werden, bevorzugt jedoch 0.1 bis 10 bar absoluter Druck, besonders bevorzugt Atmosphärendruck.

Die Katalysatormenge beim erfindungsgemäßen Verfahren liegt in der Regel bei 0.001 bis 10 mol-%, bezogen auf die Verbindung der Formel (I). Bevorzugt wird die Reaktion mit 0.1 bis 1 mol-% Katalysator durchgeführt.

Die Reaktionstemperatur ist kein kritischer Parameter. Im allgemeinen wird die Reaktionstemperatur durch den Siedepunkt des verwandten Lösungsmittels bzw. durch die Siedepunkte der Edukte bestimmt, sofern nicht durch die Einwirkung von Druck diesem entgegengewirkt wird.

Das allgemeine Reaktionsschema des erfindungsgemäßen Verfahrens ist demnach: 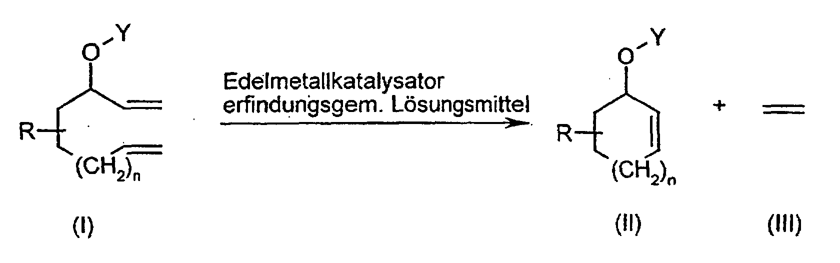 wobei die Bedeutung der Substituenten den oben aufgeführten Definitionen entspricht.

### Beispiele

### Beispiel 1: Cyclohexen-2-ol

63 mg (0.5 mmol) 3-Hydroxy-1,7-octadien und 17 mg Bis(tricyclohexylphosphin)benzylidenruthenium(IV)dichlorid (1.3 mol-%) werden in einem Schlenk-Rohr unter Argon-Atmosphäre in ml abs. Chloroform gelöst. Man läßt 14 h bei 56°C reagieren. Zur Aufarbeitung filtriert man über eine sehr kurze (ca. 0,5 cm) Kieselgel-Filtriersäule (Laufmittel: Acetonitril) und engt ein. Ausbeute an Cyclohexen-2-ol: 47 mg (0.48 mmol, 96 %). Es ist zu beachten, daß das Chloroform kein Ethanol (als Stabilisator) enthalten darf. 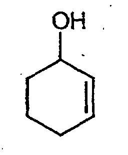 ¹H NMR (400 MHz, CDCl₃) δ 5.75 (1H, d, *J*= 10.0 Hz), 5.70 (1H, d, *J*= 10.0 Hz), 4.18 (1H, s), 3.72 (1H, s), 1.98 (2H, m), 1.84 (1H, m), 1.72 (1H, m), 1.55 (2H, m).

### Beispiel 2: Cyclohexen-2-ol

63 mg (0.5 mmol) 3-Hydroxy-1,7-octadien und 17 mg Bis(tricyclohexylphosphin)benzylidenruthenium(IV)dichlorid (1.3 mol-%) werden in einem Schlenk-Rohr unter Argon-Atmosphäre in 5 ml abs. Bromoform gelöst. Man läßt 5 h bei 80°C reagieren. Zur Aufarbeitung filtriert man über eine sehr kurze (ca. 0,5 cm) Kieselgel-Filtriersäule (Laufmittel: Acetonitril) und engt ein. Ausbeute an Cyclohexen-2-ol: 48 mg (0.49 mmol, 99 %).

## Patentansprüche

1. Verfahren zur Herstellung von zumindest α-substituierten Ringsystemen der Formel (II) 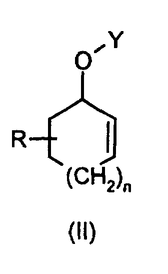 in welcher
Y Wasserstoff, Acyl, Alkyl und Aryl, Sulfonyl
R Wasserstoff darstellt,
und
n die Zahlen 1, 2, 3 oder 4 bedeuten,
in welchem eine Verbindung der Formel (I) 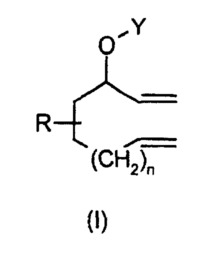 in welcher
Y, R und n die oben angegebene Bedeutung haben,
in Gegenwart eines Edelmetallkatalysators einer Metathesereaktion unterworfen wird,
**dadurch gekennzeichnet, daß** die Reaktion in einem Lösungsmittel ausgewählt aus mindestens einem Mitglied einer Gruppe bestehend aus sekundären Alkoholen, tertiären Alkoholen, Trihalogenmethanverbindungen, superkritischem Kohlendioxid und Phenylessigsäureethylester durchgeführt wird.

2. Verfahren gemäß Anspruch 1, **dadurch gekennzeichnet, daß** als Edelmetallkatalysator ein Rutheniumkomplex eingesetzt wird.

3. Verfahren gemäß Ansprüchen 1 und 2, **dadurch gekennzeichnet, daß** es sich bei dem Edelmetallkatalysator um den Rutheniumkomplex Bis(tricyclohexylphosphin)benzylidenruthenium(IV)dichlorid handelt.

4. Verfahren gemäß Ansprüchen 1 bis 3, **dadurch gekennzeichnet, daß** als Lösungsmittel eine Trihalogenmethanverbindung eingesetzt wird.

5. Verfahren gemäß Ansprüchen 1 bis 4, **dadurch gekennzeichnet, daß** als Lösungsmittel Chloroform eingesetzt wird.

6. Verfahren gemäß Ansprüchen 1 bis 4, **dadurch gekennzeichnet, daß** als Lösungsmittel Bromoform eingesetzt wird.

7. Verfahren gemäß Ansprüchen 1 bis 6, **dadurch gekennzeichnet, daß** in Formeln (I) und (II) Y Wasserstoff bedeutet und n den Wert 1 hat.

## Claims

1. Process for preparing at least α-substituted ring systems of the formula (II) 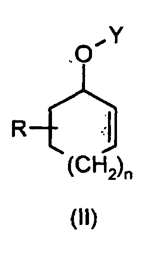 in which
Y represents hydrogen, acyl, alkyl and aryl, sulphonyl,
R represents hydrogen,
and
n represents the numbers 1, 2, 3 or 4,
in which a compound of the formula (I) 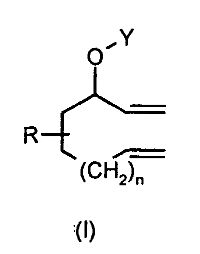 in which
Y, R and n are each as defined above,
is subjected to a metathesis reaction in the presence of a noble metal catalyst,
**characterized in that** the reaction is carried out in a solvent selected from at least one member of a group consisting of secondary alcohols, tertiary alcohols, trihalogenomethane compounds, supercritical carbon dioxide and ethyl phenylacetate.

2. Process according to Claim 1, **characterized in that** the noble metal catalyst used is a ruthenium complex.

3. Process according to Claims 1 and 2, **characterized in that** the nobel metal catalyst is the ruthenium complex bis(tricyclohexylphosphine)benzylideneruthenium(IV) dichloride.

4. Process according to Claims 1 to 3, **characterized in that** the solvent used is a trihalogenomethane compound.

5. Process according to Claims 1 to 4, **characterized in that** the solvent used is chloroform.

6. Process according to Claims 1 to 4, **characterized in that** the solvent used is bromoform.

7. Process according to Claims 1 to 6, **characterized in that**, in the formulae (I) and (II), Y represents hydrogen and n is 1.

## Revendications

1. Procédé pour la préparation de systèmes cycliques au moins α-substitués répondant à la formule (II) 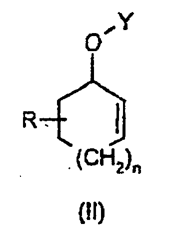 dans laquelle
Y représente un atome d'hydrogène, un groupe acyle, alkyle, aryle ou sulfonyle,
R représente un atome d'hydrogène
et
n a pour valeur 1,2 3 ou 4,
dans lequel un posé répondant à la formule (I) 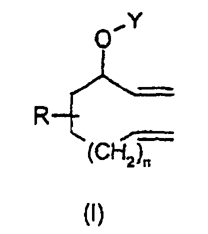 dans laquelle
Y, R et n ont les significations indiquées ci-dessus,
est soumis, en présence d'un catalyseur à base d'un métal noble, à une réaction de métathèse,
**caractérisé en ce que** cette réaction est effectuée dans un solvant choisi parmi au moins un membre d'un groupe constitué d'alcools secondaires, alcools tertiaires, posés trihalogénométhanes, dioxyde de carbone supercritique et ester éthylique de l'acide phénylacétique.

2. Procédé selon la revendication 1, **caractérisé en ce que** l'on utilise, comme catalyseur à base de métal noble, un complexe de ruthénium.

3. Procédé selon les revendications 1 et 2, **caractérisé en ce que** le catalyseur à base de métal noble est le complexe de ruthénium dichlorure de bis(tricyclohexylphosphine) benzylidèneruthénium(IV).

4. Procédé selon les revendications 1 à 3, **caractérisé en ce que** l'on utilise, comme solvant, un posé trihalogénométhane.

5. Procédé selon les revendications 1 à 4, **caractérisé en ce que** l'on utilise, comme solvant, le chloroforme.

6. Procédé selon les revendications 1 à 4, **caractérisé en ce que** l'on utilise, comme solvant, le bromoforme.

7. Procédé selon les revendications 1 à 6, **caractérisé en ce que**, dans les formules (I) et (II), Y représente un atome d'hydrogène et n a pour valeur 1.
